(19) Europäisches Patentamt
European Patent Office
Office européen des brevets

(11) EP 0 558 376 B1

(12) **FASCICULE DE BREVET EUROPEEN**

(45) Date de publication et mention de la délivrance du brevet:
**08.10.1997 Bulletin 1997/41**

(51) Int. Cl.[6]: **C07C 37/60**, C07C 39/08

(21) Numéro de dépôt: **93400388.0**

(22) Date de dépôt: **17.02.1993**

(54) **Procédé de monohydroxylation de composés phénoliques**

Verfahren zur Monohydroxylierung von Phenolverbindungen

Method of monohydroxylating phenolic compounds

(84) Etats contractants désignés:
**DE FR GB IT NL SE**

(30) Priorité: **26.02.1992 FR 9202210**
**26.02.1992 FR 9202211**

(43) Date de publication de la demande:
**01.09.1993 Bulletin 1993/35**

(73) Titulaire: **RHONE-POULENC CHIMIE**
**92408 Courbevoie Cédex (FR)**

(72) Inventeurs:
- **Costantini, Michel**
  **F-69003 Lyon (FR)**
- **Laucher, Dominique**
  **F-69007 Lyon (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 480 800** **FR-A- 2 071 464**
**FR-A- 2 266 683** **JP-A- 0 278 641**

- **CHEMICAL ABSTRACTS, vol. 113, no. 3, 16 juillet 1990, Columbus, Ohio, US; abstract no. 23364, page 606, colonne 2; & JP-A-2 078 641**

Remarques:
Le dossier contient des informations techniques présentées postérieurement au dépôt de la demande et ne figurant pas dans le présent fascicule.

EP 0 558 376 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.16/3.4

## Description

La présente invention a pour objet un procédé de monohydroxylation de composés phénoliques en vue d'obtenir des composés aromatiques dihydroxylés. L'invention vise plus particulièrement, un procédé de monohydroxylation de phénols et d'éthers de phénol, par le peroxyde d'hydrogène.

De nombreux procédés d'hydroxylation des phénols sont décrits dans l'état de la technique.

Il est décrit dans JP-A-0 278 641, un procédé de polyhydroxylation du phénol en 1,2,4-trihydroxybenzène qui consiste à oxyder le phénol par le peroxyde d'hydrogène, en présence d'un acide fort (acide sulfurique), dans un solvant polaire contenant de l'azote, notamment l'acétonitrile.

Concernant les procédés de monohydroxylation du phénol, on peut citer, entre autres, le brevet FR-A 2 071 464 qui concerne un procédé industriel très important d'hydroxylation de phénols et d'éthers de phénols.

Ledit procédé consiste à réaliser l'hydroxylation, par l'eau oxygénée, en présence d'un acide fort. Parmi ces acides forts, l'acide sulfurique, l'acide paratoluène-sulfonique, l'acide perchlorique sont les plus utilisés.

L'hydroxylation du phénol effectuée dans les conditions décrites conduit à un mélange d'hydroquinone et de pyrocatéchine, avec prédominance de celle-ci puisque le rapport hydroquinone/pyrocatéchine varie le plus souvent entre 0,3 et 0,7.

On a proposé, selon FR-A 2 266 683, un perfectionnement à ce procédé qui consiste à effectuer l'hydroxylation, en présence d'une cétone. Il en résulte une amélioration du rendement de la réaction en hydroquinone et pyrocatéchine. Toutefois, tous les exemples décrits conduisent à une quantité plus importante de pyrocatéchine par rapport à celle d'hydroquinone.

Les procédés connus conduisent donc principalement à la pyrocatéchine.

Il s'avère que pour répondre à la demande du marché qui est fluctuante, il est important, de disposer d'un procédé industriel permettant d'augmenter la production d'hydroquinone formée par rapport à la quantité de pyrocatéchine.

Afin d'apporter une solution à ce problème technique, la demanderesse a proposé dans FR-A 2 667 598, un procédé permettant d'accroître la quantité d'hydroquinone formée par rapport à la quantité de pyrocatéchine et d'obtenir dans sa variante préférentielle, plus d'hydroquinone que de pyrocatéchine.

Ledit procédé consiste à effectuer l'hydroxylation du phénol en présence d'une quantité efficace d'un acide fort, ledit procédé étant caractérisé par le fait que la réaction est conduite en présence d'un composé cétonique choisi parmi la benzophénone et les benzophénones dont les atomes d'hydrogène du noyau aromatique peuvent être substitués par un groupe électro-donneur.

Conformément au procédé décrit dans FR-A 2 667 598, la présence du composé cétonique tel que sélectionné, lors de l'hydroxylation du phénol joue sur la régiosélectivité de la réaction et des rapports hydroquinone/pyrocatéchine variant entre 1,0 et 1,13 sont avantageusement obtenus.

Poursuivant ses recherches, la demanderesse a perfectionné l'invention décrite dans FR-A 2 667 598 et trouvé que des rapports hydroquinone/ pyrocatéchine encore plus élevés pouvaient être obtenus, en ajoutant dans le mélange réactionnel une faible quantité d'un solvant organique, aprotique, présentant certaines caractéristiques de polarité et de basicité.

Plus précisement, la présente invention a pour objet un Procédé de monohydroxylation d'un composé phénolique présentant un atome d'hydrogène libre en position para, en vue de favoriser la formation d'un composé aromatique para-dihydroxylé, par réaction du composé phénolique de départ avec le peroxyde d'hydrogène, en présence d'une quantité efficace d'un acide tort et d'un composé cétonique, ledit procédé étant caractérisé par le fait que la réaction est conduite en présence d'une quantité efficace :

- d'un composé cétonique choisi parmi la benzophénone ou une benzophénone porteur d'un groupe électro-donneur,
- et d'un solvant organique aprotique polaire présentant une basicité telle qu'il possède un "nombre donneur" intérieur à 25,

et que la quantité de peroxyde d'hydrogène est telle que le rapport molaire $H_2O_2$/composé phénolique est au plus de 0,3.

Une première variante du procédé de l'invention consiste à faire appel à un solvant organique polaire et peu basique c'est-à-dire présentant une polarité telle que sa constante diélectrique est supérieure ou égale à 20 et une basicité telle qu'il possède un "nombre donneur" inférieur à 25.

Une deuxième variante du procédé de l'invention consiste à faire appel à un solvant organique moins polaire mais basique c'est-à-dire présentant une polarité telle que sa constante diélectrique est inférieure à 20 et une basicité telle qu'il possède un "nombre donneur" supérieur ou égal à 15 et inférieur à 25.

L'invention s'applique préférentiellement à l'hydroxylation du phénol en hydroquinone et pyrocatéchine.

Il a maintenant été trouvé que la présence d'un solvant ayant les caractéristiques telles que précédemment définies permettait d'accroître le rapport hydroquinone/pyrocatéchine.

Ainsi, conformément à la présente invention des rapports de l'ordre de 1,1 à 1,3 et plus, peuvent être obtenus, selon la nature du solvant organique mis en oeuvre.

Il est à noter que le choix du solvant organique à mettre en oeuvre est tout à fait critique et qu'il importe de faire appel à un solvant organique aprotique, très polaire mais peu basique ou peu polaire mais basique.

Ainsi, si l'on met en oeuvre un solvant organique aprotique de forte polarité et de forte basicité tel que le diméthylformamide ou la N-méthylpyrrolidone, on n'obtient pas l'effet recherché.

Par ailleurs, il a été constaté de manière inattendue, que cet effet de solvant choisi conformément à l'invention, intervenait quelle que soit la nature du composé cétonique.

Ainsi, il devient possible, selon le procédé de l'invention, d'adapter très facilement la quantité d'hydroquinone aux besoins du marché, par simple ajout du solvant organique adéquate.

Plusieurs impératifs président au choix du solvant organique.

Une première caractéristique du solvant organique est qu'il soit aprotique et stable dans le milieu réactionnel.

On entend par solvant aprotique, un solvant qui, dans la théorie de Lewis n'a pas de protons à libérer.

Sont exclus de la présente invention, les solvants qui ne sont pas stables dans le milieu réactionnel et qui se dégradent soit par oxydation, soit par hydrolyse. A titre d'exemples de solvants réactionnels ne convenant pas à l'invention, on peut citer les solvants de type ester dérivés des acides carboxyliques tels que notamment l'acétate de méthyle ou d'éthyle, le phtalate de méthyle ou d'éthyle, le benzoate de méthyle etc..

Les solvants organiques convenant pour la mise en oeuvre du procédé de l'invention, doivent répondre à certaines exigences au niveau de leur polarité et de leur basicité caractérisée par le nombre donneur.

Une première classe de solvants organiques convenant tout à fait bien à la mise en oeuvre du procédé de l'invention, sont les solvants organiques polaires et peu basiques.

On choisit, conformément à l'invention, un solvant organique qui présente une constante diélectrique supérieure ou égale à 20. La borne supérieure ne présente aucun caractère critique. On préfère faire appel à un solvant organique ayant une constante diélectrique élevée, de préférence comprise entre 25 et 75.

Le solvant organique présentant les caractéristiques de polarité précitées doit également satisfaire à certaines conditions de basicité. En effet, ledit solvant ne doit pas être trop basique. Pour déterminer si un solvant satisfait à cette exigence, on apprécie sa basicité en se référant au "nombre donneur". On choisit un solvant organique polaire présentant un nombre donneur inférieur à 25, de préférence inférieur ou égal à 20. La borne inférieure ne présente aucun caractère critique. On choisit, de préférence, un solvant organique ayant un nombre donneur compris entre 2 et 17.

En ce qui concerne l'autre classe de solvants revendiqués, les caractéristiques desdits solvants sont définies ci-après.

Les solvants appartenant à cette catégorie sont des solvants organiques peu polaires mais basiques.

On choisit, conformément à l'invention, un solvant organique qui présente une constante diélectrique inférieure à environ 20. La borne inférieure ne présente aucun caractère critique. On préfère faire appel à un solvant organique ayant une constante diélectrique faible, de préférence comprise entre 2 et 15.

En ce qui concerne sa basicité, elle doit être telle qu'il possède un "nombre donneur" supérieur ou égal à 15 et inférieur à 25. On choisit, de préférence, un solvant organique ayant un nombre donneur compris entre 15 et 25.

Afin de déterminer si le solvant organique répond aux conditions de constante diélectrique énoncées ci-dessus, on peut se reporter, entre autres, aux tables de l'ouvrage : Techniques of Chemistry, II - Organic solvents - p. 536 et suivantes, 3$^{ème}$ édition (1970).

Pour ce qui est des exigences concernant la basicité du solvant organique à mettre en oeuvre, on rappelera que le "nombre donneur" ou "donor number" désigné de manière abrégée DN, donne une indication sur le caractère nucléophile du solvant et révèle son aptitude à donner son doublet.

Dans l'ouvrage de Christian REINHARDT, [Solvents and Solvent Effects in Organic Chemistry - VCH p.19 (1988)], on trouve la définition du "donor number" qui est défini comme le négatif ($-\Delta H$) de l'enthalpie (Kcal/mol) de l'interaction entre le solvant et le pentachlorure d'antimoine, dans une solution diluée de dichloroéthane.

Comme exemples de solvants organiques aprotiques, polaires répondant aux caractéristiques de basicité précitées, susceptibles d'être mis en oeuvre dans le procédé de l'invention, on peut citer plus particulièrement :

- les composés nitrés tels que le nitrométhane, le nitroéthane, le nitro-1 propane, le nitro-2 propane ou leurs mélanges, le nitrobenzène,
- les nitriles alphatiques ou aromatiques comme l'acétonitrile, le propionitrile, le butanenitrile, l'isobutanenitrile, le benzonitrile, le cyanure de benzyle,
- la tétraméthylène sulfone (sulfolane),
- le carbonate de propylène.

On peut également utiliser un mélange de solvants.

Parmi les solvants précités, on met en oeuvre, préférentiellement, l'acétonitrile.

En ce qui concerne l'autre classe de solvants revendiqués, on donne, ci-après, des exemples de solvants aprotiques peu polaires et basiques susceptibles d'être mis en oeuvre dans le procédé de l'invention :

- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les esters neutres phosphoriques tels que, notamment, le phosphate de triméthyle, le phosphate de triéthyle, le phosphate de butyle, le phosphate de triisobutyle, le phosphate de tripentyle,
- le carbonate d'éthylène.

On peut également utiliser un mélange de solvants.

Conformément au procédé de l'invention, on effectue la préparation d'un composé aromatique dihydroxylé, par hydroxylation d'un composé phénolique à l'aide de peroxyde d'hydrogène.

Dans l'exposé qui suit de la présente invention, on entend par "composé aromatique", la notion classique d'aromaticité telle que définie dans la littérature, notamment par Jerry MARCH, Advanced Organic Chemistry, 3$^{ème}$ édition, John Wiley and Sons, 1985, p.37 et suivantes.

La présente invention s'applique tout particulièrement au composé phénolique de formule générale (I) :

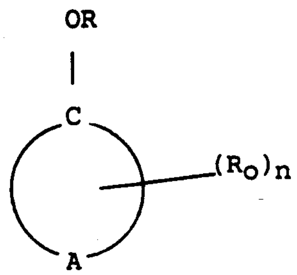

(I)

dans laquelle :

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un atome d'hydrogène ou un radical hydrocarboné ayant de 1 à 24 atomes de carbone qui peut être un radical aliphatique saturé ou insaturé, linéaire ou ramifié ou un radical cycloaliphatique saturé ou insaturé ou aromatique, monocyclique ou polycyclique,
- $R_o$ représente un ou plusieurs substituants, identiques ou différents,
- n est un nombre inférieur ou égal à 4.

Le procédé de l'invention s'applique à tout composé phénolique répondant à la formule générale (I) et, plus particulièrement, aux composés phénoliques de formule (I) dans laquelle :

- le radical R représente l'un des groupes suivants :

  . un atome d'hydrogène
  . un radical alkyle linéaire ou ramifié ayant de 1 à 6 atomes de carbone,
  . un radical cyclohexyle,
  . un radical phényle,
  . un radical benzyle,

- le ou les radicaux $R_o$ représentent l'un des groupes suivants :

  . un atome d'hydrogène
  . un radical alkyle linéaire ou ramifié ayant de 1 à 6 atomes de carbone,
  . un radical cyclohexyle,
  . un radical alcényle linéaire ou ramifié ayant de 2 à 6 atomes de carbone,
  . un radical alkoxy du type $R_1$-O-, où $R_1$ représente un radical alkyle linéaire ou ramifié ayant de 1 à 6 atomes de carbone,
  . un groupe acyle ayant de 2 à 6 atomes de carbone,

. un groupe $-COOR_2$, où $R_2$ représente un radical alkyle linéaire ou ramifié ayant de 1 à 4 atomes de carbone,
. un atome d'halogène, de préférence, de fluor, de chlore, de brome,
. un groupe $-CF_3$.

- n est un nombre égal à 0, 1, 2 ou 3.

Le composé phénolique de formule (I) peut être porteur d'un ou plusieurs substituants. Des exemples de substituants sont donnés ci-dessus mais cette liste ne présente aucun caractère limitatif. N'importe quel substituant peut être présent sur le cycle dans la mesure où il n'interfère pas au niveau du produit désiré.

Parmi les composés de formule (I), on met en oeuvre plus particulièrement ceux dont le reste (A) représente :

- un radical carbocyclique aromatique monocyclique ou polycyclique avec des cycles pouvant former entre eux un système orthocondensé répondant à la formule (Ia) :

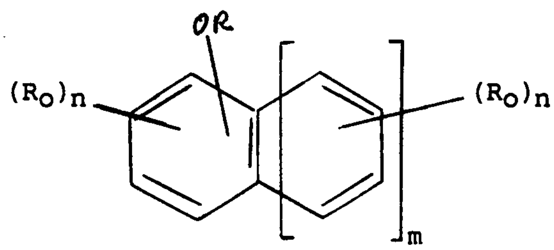

(Ia)

dans ladite formule (Ia), m représente un nombre égal à 0, 1 ou 2 et les symboles $R_o$ et n identiques ou différents ayant la signification donnée précédemment,
- un radical constitué par un enchaînement de deux ou plusieurs radicaux carbocycliques aromatiques monocycliques répondant à la formule (Ib) :

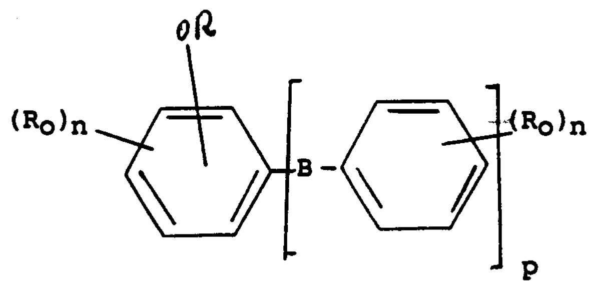

(Ib)

dans ladite formule (Ib), les symboles $R_o$ et n identiques ou différents ont la signification donnée précédemment, p est un nombre égal à 0, 1, 2 ou 3 et B représente :
- un lien valentiel
- un radical alkylène ou alkylidène ayant de 1 à 4 atomes de carbone, de préférence, un radical méthylène ou isopropylidène,
- un des groupes suivants :
—O—, —COO—, —OCOO—,
$-SO_2-$,

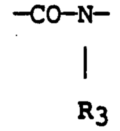 ,

dans ces formules, $R_3$ représente un atome d'hydrogène ou un radical alkyle ayant de 1 à 4 atomes de carbone, un radical cyclohexyle ou phényle.

Les composés de formule (I) mis en oeuvre préférentiellement répondent aux formules (Ia) et (Ib) dans lesquelles :

- $R_o$ représente un atome d'hydrogène, un radical alkyle ou alkoxy linéaire ou ramifié ayant de 1 à 6 atomes de carbone, de préférence, de 1 à 4 atomes de carbone un radical cyclohexyle, un radical phényle,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

L'invention s'applique plus particulièrement aux composés phénoliques de formule générale (I'):

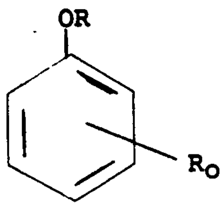

(I')

dans laquelle R et $R_o$, identiques ou différents représentent un atome d'hydrogène, un radical alkyle ayant de 1 à 4 atomes de carbone, un radical cyclohexyle, un radical phényle.

Encore plus préférentiellement, on choisit les composés de formule (I') dans laquelle R représente un atome d'hydrogène et $R_o$ représente un atome d'hydrogène, un radical méthyle, un radical méthoxy.

A titre illustratif de composés phénoliques de formule (I) susceptibles d'être mis en oeuvre dans le procédé de l'invention, on peut mentionner plus particulièrement :

- ceux dans lesquels le reste A répond à la formule (Ia) dans laquelle m et n sont égaux à O, tels que le phénol ou l'anisole,
- ceux dans lesquels le reste A répond à la formule (Ia) dans laquelle m est égal à O et n est égal à 1, tels que l'orthocrésol, le métacrésol, le méthoxy-2 phénol, l'éthyl-2 phénol, l'éthyl-3 phénol, le propyl-2 phénol, le sec-butyl-2 phénol, le tert-butyl-2 phénol, le tert-butyl-3 phénol, le méthoxy-2 phénol, le méthoxy-3 phénol, le salicylate de méthyle, le chloro-2 phénol, le chloro-3 phénol,
- ceux dans lesquels le reste A répond à la formule (Ia) dans laquelle m est égal à O et n est égal à 2, tels que le diméthyl-2,3 phénol, le diméthyl-2,5 phénol, le diméthyl-2,6 phénol, le diméthyl-3,5 phénol, le dichloro-2,3 phénol,le dichloro-2,5 phénol,le dichloro-2,6 phénol,le dichloro-3,5 phénol,
- ceux dans lesquels le reste A répond à la formule (Ia) dans laquelle m est égal à 0 et n est égal à 3, tels que le triméthyl-2,3,5 phénol, le triméthyl-2,3,6 phénol, le di-tert butyl-2,6 phénol, le di-tert butyl-3,5 phénol, le trichloro-2,3,5 phénol, le trichloro-2,3,6 phénol,
- ceux dans lesquels le reste A répond à la formule (Ia) dans laquelle m est égal à 1 et n est égal à 1, tels que l'hydroxy-1 naphtalène,
- ceux dans lesquels le reste A répond à la formule (Ib) dans laquelle n est supérieur ou égal à 1, tels que le phénoxy-2 phénol, le phénoxy-3 phénol.

Le présent procédé convient tout particulièrement bien à la préparation d'hydroquinone et de pyrocatéchol à partir du phénol.

Conformément au procédé de l'invention, on fait intervenir un solvant organique aprotique polaire au cours du procédé de monohydroxylation du composé phénolique de formule (I) effectuée en présence d'un acide fort et d'un composé cétonique.

Etant donné que l'effet de solvant est constaté quelle que soit la nature du composé cétonique mis en oeuvre, il est donc possible de mettre en oeuvre tout composé cétonique.

On met en oeuvre préférentiellement, les composés cétoniques qui présentent eux-mêmes un effet para-orienteur tel que mis en évidence dans FR-A 2 667 598.

Ainsi, on fait appel tout particulièrement aux composés cétoniques répondant à la formule générale (IIa) :

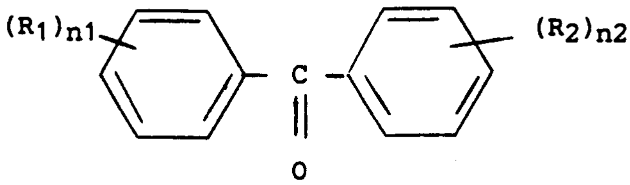

(IIa)

dans ladite formule (IIa)

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position $\alpha$ par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

Comme mentionné précédemment, lesdits composés cétoniques font l'objet de la demande de brevet FR-A 2 667 598.

On entend par "groupe électro-donneur", un groupe tel que défini par H.C. BROWN dans l'ouvrage de Jerry MARCH - Advanced Organic Chemistry, chapitre 9, pages 243 et 244 (1985).

Le groupe électro-donneur est choisi de telle sorte qu'il ne réagisse pas dans les conditions d'acidité de l'invention.

Des exemples de groupes électro-donneurs convenant bien à l'invention décrite dans FR-A 2 667 598, sont les suivants :

- les radicaux alkyle linéaires ou ramifiés ayant de 1 à 4 atomes de carbone,
- le radical phényle,
- les radicaux alkoxy $R_3$-O- dans lesquels $R_3$ représente un radical alkyle linéaire ou ramifié ayant de 1 à 4 atomes de carbone ou le radical phényle,
- le groupe hydroxyle,
- l'atome de fluor.

Comme exemples de composés cétoniques particulièrement adaptés à l'invention décrite dans FR-A 2 667 598, on peut citer tout particulièrement les composés cétoniques répondant à la formule générale (II) dans laquelle $R_1$ et $R_2$, identiques ou différents représentent un atome d'hydrogène ou un groupe électro-donneur, de préférence en position 4,4' et $n_1$, $n_2$ identiques ou différents sont égaux à 0 ou 1.

On fait appel préférentiellement aux composés cétoniques répondant à la formule (II) dans laquelle $R_1$ et $R_2$ identiques ou différents représentent un atome d'hydrogène ; un radical méthyle, éthyle, tertiobutyle, phényle ; un radical méthoxy ou éthoxy ; un groupe hydroxyle, de préférence en position 3,3' ou 4,4'.

Comme exemples spécifiques de cétones qui peuvent être utilisées dans le procédé de l'invention décrit dans FR-A 2 667 598, on peut citer plus particulièrement :

- la benzophénone
- la méthyl-2 benzophénone
- la diméthyl-2,4 benzophénone
- la diméthyl-4,4' benzophénone
- la diméthyl-2,2' benzophénone
- la diméthoxy-4,4' benzophénone
- la fluorénone
- l'hydroxy-4 benzophénone
- la dihydroxy-4-4' benzophénone
- le benzoyl-4 biphényle

Selon le procédé de l'invention, intervient un solvant organique aprotique polaire au cours du procédé de monohydroxylation du composé phénolique de formule (I) effectuée en présence d'un acide fort et d'un composé cétonique.

La quantité de solvant organique à mettre en oeuvre est déterminée en fonction de la nature du solvant organique choisi.

Ainsi, lors de la mise en oeuvre d'un solvant organique polaire mais peu basique, elle est déterminée de telle sorte

que le rapport molaire entre le nombre de moles de solvant organique et le nombre de moles de composé phénolique de formule (I) varie entre 0,1 et 2,0, de préférence, entre 0,25 et 1,0.

Si l'on fait appel à un solvant organique peu polaire et basique, la quantité mise en oeuvre est déterminée de telle sorte que le rapport molaire entre le nombre de moles de solvant organique et le nombre de moles de composé phénolique de formule (I) varie entre 0,01 et 0,25, de préférence, entre 0,025 et 0,15.

D'une manière générale, on choisit la quantité de solvant à ajouter en fonction de la basicité du solvant. La quantité de solvant mise en oeuvre sera d'autant plus faible que sa basicité sera plus forte. En d'autres termes, une quantité située vers la limite inférieure de l'intervalle précédemment défini sera choisie lorsque le solvant présente une basicité élevée.

Le composé cétonique de formule (II) qui a été précédemment définie intervient en une quantité définie ci-après.

Généralement, la quantité du composé cétonique de formule (II) exprimée en moles par mole de peroxyde d'hydrogène varie entre $1.10^{-3}$ mole et 10. Il n'est pas nécessaire de dépasser 1,0 mole de composé cétonique par mole de peroxyde d'hydrogène. Dans la pratique, la quantité de composé cétonique est le plus souvent comprise entre 0,05 et 1,0 mole par mole de peroxyde d'hydrogène.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de $H_2O_2$ et, de préférence, aux environs de 70 %.

La quantité de peroxyde d'hydrogène peut aller jusqu'à 1 mole de $H_2O_2$ pour 1 mole de composé phénolique de formule (I).

Il est cependant préférable pour obtenir un rendement industriellement acceptable d'utiliser un rapport molaire peroxyde d'hydrogène/composé phénolique de formule (I) de 0,01 à 0,3 et, de préférence, de 0,05 à 0,10.

Afin d'avoir une vitesse de réaction suffisante, on limite la teneur initiale du milieu en eau à 20 % en poids et, de préférence, à 10 % en poids.

Les teneurs pondérales indiquées sont exprimées par rapport au mélange composé phénolique de formule (I)/peroxyde d'hydrogène/eau.

Cette eau initiale correspond à l'eau introduite avec les réactifs et notamment avec le peroxyde d'hydrogène.

Intervient dans le procédé de l'invention, un acide fort. Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation par le peroxyde d'hydrogène.

On peut citer plus particulièrement les oxyacides halogénés ou non tels que l'acide sulfurique, l'acide pyrosulfurique, l'acide perchlorique, l'acide nitrique, les acides halogénosulfoniques tels que l'acide fluorosulfonique, l'acide chlorosulfonique ou l'acide trifluorométhanésulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide perchlorique, l'acide trifluorométhanesulfonique, l'acide paratoluènesulfonique, l'acide chlorosulfonique, l'acide fluorosulfonique, l'acide méthanesulfonique.

On choisit, tout particulièrement, l'acide perchlorique ou l'acide trifluorométhanesulfonique.

La quantité d'acide exprimée par le rapport du nombre d'équivalents de protons au nombre de mole de peroxyde d'hydrogène peut varier entre environ $1.10^{-4}$ et environ 1,0.

Une variante préférée du procédé de l'invention consiste à choisir un rapport $H^+/H_2O_2$ compris entre $1.10^{-3}$ et 0,1.

Une variante préférée du procédé de l'invention consiste à ajouter un agent complexant des ions métalliques présents dans le milieu car ceux-ci sont préjudiciables au bon déroulement du procédé de l'invention, notamment dans le cas des phénols où les rendements en produits d'hydroxylation sont faibles. Par conséquent, il est préférable d'inhiber l'action des ions métalliques.

Les ions métalliques néfastes au déroulement de l'hydroxylation sont des ions de métaux de transition et plus particulièrement, les ions fer, cuivre, chrome, cobalt, manganèse et vanadium.

Les ions métalliques sont apportés par les réactifs et notamment les composés aromatiques et l'appareillage utilisé. Pour inhiber l'action de ces ions métalliques, il suffit de conduire la réaction en présence d'un ou plusieurs agents complexants stables vis-à-vis du peroxyde d'hydrogène et donnant des complexes ne pouvant être décomposés par les acides forts présents et dans lesquels le métal ne peut plus exercer d'activité chimique.

A titre d'exemples non limitatifs d'agents complexants, on peut faire appel, notamment, aux divers acides phosphoriques tels que, par exemple, l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide

phosphonique, l'acide éthylphosphonique, l'acide phénylphosphonique.

On peut également mettre en oeuvre les esters des acides précités et l'on peut mentionner, plus particulièrement, les orthophosphates de mono- ou dialkyle, de mono-ou dicycloalkyle, de mono- ou dialkylaryle, par exemple, le phosphate d'éthyle ou de diéthyle, le phosphate d'hexyle, le phosphate de cyclohexyle, le phosphate de benzyle.

La quantité d'agent complexant dépend de la teneur du milieu réactionnel en ions métalliques.

La quantité d'agent complexant exprimée en nombres de moles d'agent complexant par mole de peroxyde d'hydrogène varie avantageusement entre 0,0001 et 0,01.

Une autre variante d'exécution du procédé de l'invention consiste à conduire le procédé de monohydroxylation de composés phénoliques de formule générale (I), au moyen du peroxyde d'hydrogène, en présence d'une quantité efficace d'un sel de métal alcalin ou alcalino-terreux d'un acide fort, d'une quantité efficace d'au moins un oxacide du phosphore, d'une quantité efficace d'au moins un composé cétonique répondant à la formule générale (II), ledit procédé étant caractérisé par le fait que la réaction est conduite en présence d'un solvant aprotique polaire tel que précédemment défini.

Par sel d'acide fort, on entend un acide présentant un pka dans l'eau inférieur à - 0,1 et, de préférence, inférieur à - 1,0.

Parmi les sels des acides répondant à cette définition, il est préférable d'utiliser les sels de métaux alcalins ou alcalino-terreux des acides stables vis-à-vis de l'oxydation par le peroxyde d'hydrogène.

Ainsi, les sels de métaux alcalins ou alcalino-terreux des acides forts précités conviennent tout-à-fait bien.

Par sels de métaux alcalins, on entend dans le présent texte les sels neutres des acides définis précédemment de lithium, de sodium, de potassium, de rubidium et de césium.

On préfère le plus souvent utiliser les sels de sodium ou de potassium et encore plus préférentiellement, pour des raisons économiques, les sels de sodium.

Parmi ces différents sels, on peut citer, parmi les préférés, le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

Par sels de métaux alcalino-terreux, on entend dans le présent texte les sels neutres des acides définis précédemment de béryllium, de magnésium, de calcium, de strontium et de baryum.

On préfère le plus souvent utiliser les sels de magnésium, de calcium et de baryum.

Parmi ces différents sels de métaux alcalino-terreux, on mettra en oeuvre, préférentiellement, le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le paratoluènesulfonate de calcium, le paratoluènesulfonate de magnésium, le fluorosulfonate de calcium, le fluorosulfonate de magnésium, le méthanesulfonate de calcium, le méthanesulfonate de magnésium.

On peut utiliser des mélanges de plusieurs sels de métaux alcalins ou alcalino-terreux.

On peut également préparer in-situ les sels de métaux alcalins ou alcalino-terreux par exemple en chargeant des quantités stoechiométriques d'acide et d'oxyde ou hydroxyde de ces métaux.

Les oxacides du phosphore sont plus particulièrement les composés à fonction acide du phosphore au degré d'oxydation 5.

On peut également utiliser des composés à fonction acide du phosphore au degré d'oxydation 3, qui seront oxydés dans le milieu par le peroxyde d'hydrogène en composés correspondants du phosphore V ; mais cela ne présente pas d'intérêt particulier, tout en ayant l'inconvénient de consommer une partie du peroxyde d'hydrogène.

Parmi ces oxacides du phosphore V, on peut citer, par exemple, l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

Les plus couramment utilisés sont, pour des questions pratiques et économiques, l'acide orthophosphorique, l'acide pyrophosphorique et l'acide (hydroxy-1 éthylidène)diphosphonique.

La quantité de sel de métal alcalin ou alcalino-terreux utilisée dans le procédé de l'invention peut varier dans de larges limites.

Généralement, cette quantité est exprimée en rapport molaire sel de métal alcalin ou alcalino-terreux/peroxyde d'hydrogène. Ce rapport est le plus souvent compris entre 0,001 et 0,10 et, de préférence, entre 0,005 et 0,05.

La quantité d'oxacide du phosphore exprimée en rapport molaire oxacide du phosphore/peroxyde d'hydrogène est le plus souvent comprise entre 0,001 et 0,20 et de préférence entre 0,05 et 0,10.

En ce qui concerne les conditions de mise en oeuvre du peroxyde d'hydrogène et du composé cétonique répondant à la formule (II), celles-ci correspondent à ce qui est décrit précédemment.

Conformément au procédé de l'invention, on réalise l'hydroxylation du composé phénolique de formule (I) à une température qui peut être comprise entre 45°C et 150°C.

Une variante préférée du procédé de l'invention consiste à choisir la température entre 45°C et 75°C.

La réaction est conduite avantageusement sous pression atmosphérique.

D'un point de vue pratique, le procédé selon l'invention est simple à mettre en oeuvre de façon continue ou discontinue.

D'un manière préférée, on choisit l'ordre des réactifs suivants : on introduit le composé phénolique de formule (I), le solvant organique aprotique polaire, éventuellement l'agent complexant, l'acide fort puis le composé cétonique de formule (II).

On porte le milieu réactionnel à la température désirée puis, l'on ajoute la solution de peroxyde d'hydrogène, de manière progressive.

En fin de réaction, le composé phénolique non transformé, et le composé cétonique de formule (II), sont séparés des produits d'hydroxylation par les moyens usuels, notamment, par distillation et sont renvoyés dans la zone réactionnelle.

On donne ci-après des exemples de réalisation de l'invention.

Les exemples qui suivent, illustrent l'invention sans toutefois la limiter.

Les exemples 1 à 21 illustrent la mise en oeuvre d'un solvant organique, aprotique, polaire et peu basique.

Les exemples 22 à 35 exemplifient la mise en oeuvre d'un solvant organique aprotique, peu polaire et basique.

Les essais a à p sont des exemples comparatifs.

Dans les exemples, les abréviations suivantes signifient :

$$TT = \frac{\text{nombre de moles de peroxyde d'hydrogène transformées}}{\text{nombre de moles de peroxyde d'hydrogène introduites}} \%$$

$$RT_{HQ} = \frac{\text{nombre de moles d'hydroquinone formées}}{\text{nombre de moles de peroxyde d'hydrogène transformées}} \%$$

$$RT_{PC} = \frac{\text{nombre de moles de pyrocatéchine formées}}{\text{nombre de moles de peroxyde d'hydrogène transformées}} \%$$

EXEMPLES

On donne, ci-après, le protocole opératoire qui va être suivi dans tous les exemples.

Dans un ballon en verre de 100 ml muni d'une agitation centrale, d'un réfrigérant, d'une ampoule de coulée et d'un thermomètre, on charge :

- 47 g (0,50 mol) de phénol,
- x g d'un composé cétonique de formule (II).

On introduit ensuite y g de solvant aprotique et z g d'acide fort (acide perchlorique ou sulfurique).

Les différentes quantités (x, y et z) peuvent être déterminées à partir des indications données dans les tableaux récapitulatifs.

On porte le mélange réactionnel à la température réactionnelle choisie 75°C (sauf indication contraire), tout en le maintenant sous agitation à 1200 tours/mn.

On introduit par l'intermédiaire de l'ampoule de coulée, en 2 minutes, la solution aqueuse de peroxyde d'hydrogène à 70,5 % en poids en une quantité également précisée dans les tableaux suivants.

On maintient le mélange réactionnel sous agitation, à 75°C, pendant la durée mentionnée dans les tableaux suivants.

On refroidit alors le mélange réactionnel et l'on effectue le dosage des produits de réaction : le peroxyde d'hydrogène résiduel est dosé par iodométrie et les diphénols formés sont dosés par chromatographie liquide, haute performance.

EXEMPLES 1 A 4

Essais comparatifs a à e :

Dans cette série d'exemples, on a mis en oeuvre deux solvants choisis conformément à l'invention, à savoir :

- le sulfolane (tétraméthylène sulfone) : exemples 1 et 2,
- le carbonate de propylène : exemples 3 et 4.

Les essais sont conduits selon le protocole opératoire précédemment défini.

Toutes les conditions et les résultats obtenus sont rassemblés dans le tableau (I) suivant :

Tableau I

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/ $H_2O_2$] | Solvant organique [rapport molaire solvant organique/ phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 1 | benzophénone (0,97) | sulfolane (0,249) | $5,2.10^{-2}$ | $1,2.10^{-2}$ | 37 mn | 92 | 46,5 | 40,5 | 1,15 |
| 2 | benzophénone (0,985) | sulfolane (0,498) | $5,05.10^{-2}$ | $1,25.10^{-2}$ | 60 mn | 100 | 49,0 | 37,5 | 1,31 |
| 3 | benzophénone (0,98) | carbonate de propylène (0,25) | $5,1.10^{-2}$ | $1,25.10^{-2}$ | 35 mn | 100 | 45,5 | 41,0 | 1,11 |
| 4 | benzophénone (0,99) | carbonate de propylène (0,495) | $5,0.10^{-2}$ | $1,35.10^{-2}$ | 45 mn | 98,5 | 45 | 37,5 | 1,20 |
| a | benzophénone (1,0) | sans | $4,9.10^{-2}$ | $1,35.10^{-2}$ | 3 mn | 100 | 43 | 42 | 1,02 |
| b | sans | sulfolane (0,245) | $5,0.10^{-2}$ | $1,25.10^{-2}$ | 150 mn | 98 | 34,5 | 45 | 0,77 |
| c | sans | carbonate de propylène (0,245) | $5,2.10^{-2}$ | $1,25.10^{-2}$ | 60 mn | 98 | 38 | 49,5 | 0,77 |
| d | benzophénone (1,0) | diméthylformamide (0,25) | $5,0.10^{-2}$ | $1,50.10^{-2}$ | 120 mn | 21,5 | 16 | 33,5 | 0,48 |
| e | benzophénone (0,98) | hexaméthylènephosphoramide (0,115) | $5,1.10^{-2}$ | $1,25.10^{-2}$ | 120 mn | 9,0 | 0,5 | 3,5 | 0,14 |
| f | benzophénone (0,98) | méthanol (0,24) | $5,1.10^{-2}$ | $1,25.10^{-2}$ | 85 mn | 100 | 37 | 39 | 0,95 |

A titre comparatif, on donne les résultats obtenus lorsque l'on conduit le procédé de l'invention :

- essai a : en l'absence d'un solvant organique,
- essai b et c : en l'absence de benzophénone mais en présence d'un solvant organique respectivement, le sulfolane et le carbonate de propylène,
- essai d et e : en présence d'un solvant organique ayant une forte constante diélectrique tel que le diméthylformamide (essai d) et l'hexaméthylènephosphoramide (essai e),

- essai f : en présence de benzophénone et en présence d'un solvant protique tel que le méthanol.

L'examen du tableau (I) met nettement en évidence que la présence d'un solvant organique polaire tel que défini selon l'invention favorise la formation de l'hydroquinone.

EXEMPLES 5 A 12

Essai comparatif g :

Dans cette série d'exemples, on a mis en oeuvre les solvants suivants choisis conformément à l'invention, à savoir :

- l'acétonitrile : exemples 5 à 8,
- le butyronitrile : exemple 9,
- le benzonitrile : exemple 10,
- le nitrométhane : exemple 11,
- le nitrobenzène : exemple 12.

Les essais sont conduits selon le protocole opératoire précédemment défini.
Toutes les conditions et les résultats obtenus sont rassemblés dans le tableau (II) suivant :

Tableau II

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/$H_2O_2$] | Solvant organique [rapport molaire solvant organique/phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 5 | benzophénone (0,96) | acétonitrile (0,25) | $5,20.10^{-2}$ | $1,5.10^{-2}$ | 35 mn | 100 | 44,5 | 40,5 | 1,10 |
| 6 | benzophénone (1,0) | acétonitrile (0,50) | $5,0.10^{-2}$ | $1,45.10^{-2}$ | 40 mn | 100 | 47 | 37,5 | 1,25 |
| 7 | benzophénone (0,49) | acétonitrile (0,75) | $5,10.10^{-2}$ | $0,65.10^{-2}$ | 250 mn | 97,5 | 47,5 | 39,5 | 1,20 |
| 8 | benzophénone (1,0) | acétonitrile (1,00) | $5,15.10^{-2}$ | $1,25.10^{-2}$ | 120 mn | 98 | 48,5 | 36,5 | 1,33 |
| 9 | benzophénone (0,98) | butyronitrile (0,25) | $5,05.10^{-2}$ | $1,15.10^{-2}$ | 60 mn | 100 | 45,5 | 40,5 | 1,12 |
| 10 | benzophénone (0,98) | benzonitrile (0,23) | $4,70.10^{-2}$ | $1,10.10^{-2}$ | 60 mn | 100 | 45,5 | 40,5 | 1,12 |
| 11 | benzophénone (1,0) | nitrométhane (0,24) | $4,8.10^{-2}$ | $1,30.10^{-2}$ | 15 mn | 100 | 41,5 | 38,0 | 1,09 |
| 12 | benzophénone (0,98) | nitrobenzène (0,25) | $5,2.10^{-2}$ | $1,35.10^{-2}$ | 30 mn | 100 | 43,5 | 39,0 | 1,12 |
| a | benzophénone (1,0) | sans | $4,9.10^{-2}$ | $1,35.10^{-2}$ | 3 mn | 100 | 43 | 42 | 1,02 |
| g | sans | acétonitrile (0,25) | $5,0.10^{-2}$ | $1,30.10^{-2}$ | 120 mn | 98 | 38 | 44,5 | 0,85 |

A titre comparatif, on donne les résultats obtenus lorsque l'on conduit le procédé de l'invention :

- essai a : en l'absence d'un solvant organique,
- essai g : en l'absence de benzophénone mais en présence d'un solvant organique l'acétonitrile.

Il ressort de l'analyse du tableau (II) que la présence d'un solvant organique polaire tel que défini selon l'invention favorise la formation de l'hydroquinone.

EXEMPLES 13 ET 14

Dans ces exemples, on a reproduit le protocole opératoire tel que donné précédemment à la différence près que la température réactionnelle est de 45°C dans l'exemple 13 et de 100°C dans l'exemple 14.

Toutes les conditions et les résultats obtenus sont rassemblés dans le tableau (III) suivant :

Tableau III

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/$H_2O_2$] | Solvant organique [rapport molaire solvant organique/phénol] | Rapport molaire $H_2O_2$/PHENOL | Rapport molaire $HClO_4/H_2O_2$ | Température °C | Durée | TT | $RT_{HQ}$ | $RT_{PC}$ | Rapport HQ/PC |
|---|---|---|---|---|---|---|---|---|---|---|
| 13 | benzophénone (0,985) | sulfolane (0,25) | $5{,}05.10^{-2}$ | $1{,}25.10^{-2}$ | 45°C | 2 h 30 | 98 | 49,5 | 36,0 | 1,38 |
| 14 | benzophénone (0,955) | sulfolane (0,25) | $5{,}2.10^{-2}$ | $1{,}1.10^{-2}$ | 100°C | 10 mn | 99,5 | 44,5 | 37,5 | 1,19 |

EXEMPLES 15 et 16 :

Dans ces exemples, on a reproduit le protocole opératoire tel que donné précédemment à la différence près que

l'acide perchlorique est remplacé par l'acide sulfurique.

Toutes les conditions et les résultats obtenus sont rassemblés dans le tableau (IV) suivant :

Tableau IV

**HYDROXYLATION DU PHENOL PAR $H_2O_2/H_2SO_4$/COMPOSE CETONIQUE DE FORMULE (II)**

| Réf. | Composé cétonique (II) [rapport molaire composé cétonique (II) /$H_2O_2$] | Solvant organique [rapport molaire solvant organique/ phénol] | Rapport molaire $H_2O_2$/PHENOL | Rapport molaire $H_2SO_4/H_2O_2$ | Durée | TT | $RT_{HQ}$ | $RT_{PC}$ | Rapport HQ/PC |
|---|---|---|---|---|---|---|---|---|---|
| 15 | benzophénone (1,0) | acétonitrile (0,25) | $5,0.10^{-2}$ | $1,3.10^{-2}$ | 60 mn | 100 | 48 | 42 | 1,14 |
| 16 | benzophénone (0,99) | sulfolane (0,485) | $4,9.10^{-2}$ | $1,55.10^{-2}$ | 60 mn | 98,5 | 46,5 | 39 | 1,19 |

EXEMPLES 17 ET 18 :

Dans les deux exemples suivants, on a fait appel à un composé cétonique différent par rapport aux exemples précédents.

Il s'agit de benzophénones substituées par un groupe électro-donneur :

- la diméthoxy-4,4' benzophénone : exemple 17,
- la diméthyl-4,4' benzophénone : exemple 18.

Toutes les conditions et les résultats obtenus sont rassemblés dans le tableau (V) suivant :

Tableau V

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/$H_2O_2$] | Solvant organique [rapport molaire solvant organique/phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 17 | diméthoxy-4,4' benzophénone (0,925) | acétonitrile (0,255) | $5{,}4.10^{-2}$ | $1{,}4.10^{-2}$ | 1 h 45 | 99,5 | 45 | 37,5 | 1,2 |
| 18 | diméthyl-4,4' benzophénone (1,0) | acétonitrile (0,25) | $4{,}95.10^{-2}$ | $1{,}7.10^{-2}$ | 32 mn | 100 | 51 | 39,5 | 1,29 |

On note l'obtention de bons rendements réactionnels et des rapports hydroquinone/pyrocatéchine élevés.

EXEMPLES 19 A 21 :

Essais comparatifs h et i :

Dans les deux exemples suivants, on a fait appel à un composé cétonique différent par rapport aux exemples précédents.

Il s'agit des composés cétoniques répondant à la formule (II) suivants :

- la pentanone-2 : exemple 19,
- l'acétophénone : exemples 20 et 21.

On donne, à titre comparatif, les résultats des essais en l'absence de solvant organique.

Toutes les conditions et les résultats obtenus sont rassemblés dans le tableau (VI) suivant :

Tableau VI

HYDROXYLATION DU PHENOL PAR $H_2O_2/HClO_4$/COMPOSE CETONIQUE DE FORMULE (II)

| Ref. | Composé cétonique (II) [rapport molaire composé cétonique (II)/$H_2O_2$] | Solvant organique [rapport molaire solvant organique/phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 19 | pentanone-2 (1,0) | acétonitrile (0,50) | $5,1.10^{-2}$ | $1,15.10^{-2}$ | 5 mn | 100 | 41,5 | 44,5 | 0,93 |
| 20 | acétophénone (1,0) | acétonitrile (0,25) | $5,0.10^{-2}$ | $1,25.10^{-2}$ | 10 mn | 100 | 37 | 48 | 0,77 |
| 21 | acétophénone (0,96) | sulfolane (0,25) | $5,15.10^{-2}$ | $1,35.10^{-2}$ | 15 mn | 100 | 38,5 | 47 | 0,82 |
| h | pentanone-2 (1,0) | sans | $5,1.10^{-2}$ | $1,35.10^{-2}$ | 3 mn | 100 | 36 | 50,5 | 0,71 |
| i | acétophénone (1,0) | sans | $5,1.10^{-2}$ | $1,25.10^{-2}$ | 2 mn | 100 | 34,5 | 49 | 0,70 |

La comparaison des exemples 19, 20, 21 et des essais comparatifs h et il permet de mettre en évidence que l'addition d'un solvant organique permet d'accroître la quantité d'hydroquinone formée, quelle que soit la nature de la cétone mise en oeuvre.

EXEMPLES 22 A 31 :

Essais comparatifs j à m :

Dans cette série d'exemples, on a mis en oeuvre différents solvants choisis conformément à l'invention, à savoir :

- l'oxyde de di n-propyle : exemple 22,
- le méthyltertiobutyléther : exemples 23 et 24,
- le diméthoxy-1,2 éthane : exemple 25,
- le tétrahydrofuranne : exemples 26 et 27,
- le dioxane : exemples 28 et 29,
- le phosphate de triéthyle : exemple 30,
- le phosphate de tributyle : exemple 31.

Les exemples sont conduits selon le protocole opératoire précédemment défini.
Toutes les conditions et les résultats obtenus sont rassemblés dans le tableau (V) suivant :

Tableau V

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/ $H_2O_2$] | Solvant organique [rapport molaire solvant organique/ phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 22 | benzophénone (0,97) | oxyde de di n-propyle (0,25) | $5,25.10^{-2}$ | $1,1.10^{-2}$ | 100 mn | 99,5 | 45 | 42 | 1,07 |
| 23 | benzophénone (0,91) | méthyltertiobutyléther (0,025) | $5,5.10^{-2}$ | $1,3.10^{-2}$ | 40 mn | 100 | 45 | 42 | 1,07 |
| 24 | benzophénone (1,0) | méthyltertiobutyléther (0,062) | $5,0.10^{-2}$ | $1,2.10^{-2}$ | 60 mn | 100 | 40 | 35,5 | 1,13 |
| 25 | benzophénone (0,99) | diméthoxy-1,2 éthane (0,12) | $5,0.10^{-2}$ | $1,15.10^{-2}$ | 45 mn | 99,5 | 46 | 42,5 | 1,08 |
| 26 | benzophénone (0,97) | tétrahydrofuranne (0,12) | $4,95.10^{-2}$ | $1,15.10^{-2}$ | 90 mn | 100 | 41 | 38,5 | 1,06 |
| 27 | benzophénone (0,96) | tétrahydrofuranne (0,25) | $5,2.10^{-2}$ | $1,15.10^{-2}$ | 110 mn | 100 | 44,5 | 40,5 | 1,10 |
| 28 | benzophénone (0,97) | dioxane (0,12) | $4,9.10^{-2}$ | $1,3.10^{-2}$ | 60 mn | 100 | 45 | 42,5 | 1,06 |
| 29 | benzophénone (0,95) | dioxane (0,25) | $5,3.10^{-2}$ | $1,25.10^{-2}$ | 120 mn | 100 | 48 | 41,0 | 1,17 |
| 30 | benzophénone (1,0) | phosphate de triéthyle (0,083) | $5,25.10^{-2}$ | $1,40.10^{-2}$ | 60 mn | 100 | 42,5 | 37,5 | 1,13 |
| 31 | benzophénone (1,0) | phosphate de tributyle (0,025) | $5,0.10^{-2}$ | $1,15.10^{-2}$ | 60 mn | 100 | 45 | 41,5 | 1,08 |

Tableau V (suite)

**HYDROXYLATION DU PHENOL PAR $H_2O_2/HClO_4$/COMPOSE CETONIQUE DE FORMULE (II)**

| Réf. | Composé cétonique (II) [rapport molaire composé cétonique (II)/ $H_2O_2$] | Solvant organique [rapport molaire solvant organique/ phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| j | benzophénone (1,0) | sans | $4,9.10^{-2}$ | $1,35.10^{-2}$ | 3 mn | 100 | 43 | 42 | 1,02 |
| k | benzophénone (1,0) | diméthylformamide (0,25) | $5,0.10^{-2}$ | $1,50.10^{-2}$ | 120 mn | 21,5 | 16 | 33,5 | 0,48 |
| l | benzophénone (0,98) | hexaméthylènephosphoramide (0,115) | $5,1.10^{-2}$ | $1,25.10^{-2}$ | 120 mn | 9,0 | 0,5 | 3,5 | 0,14 |
| m | benzophénone (0,98) | méthanol (0,24) | $5,1.10^{-2}$ | $1,25.10^{-2}$ | 85 mn | 100 | 37 | 39 | 0,95 |

A titre comparatif, on donne les résultats obtenus lorsque l'on conduit le procédé de l'invention :

- essai j : en l'absence d'un solvant organique,
- essai k et l : en présence de benzophénone et en présence d'un solvant organique ayant une forte constante diélectrique tel que le diméthylformamide (essai k) et l'hexaméthylènephosphoramide (essai l),

- essai m : en présence de benzophénone et d'un solvant protique tel que le méthanol.

L'examen du tableau (V) met nettement en évidence que la présence d'un solvant organique tel que défini selon l'invention favorise la formation de l'hydroquinone.

EXEMPLES 32 et 33 :

Essais comparatifs n et o :

On effectue une série d'essais en faisant appel, comme solvant organique, à l'oxyde de diisopropyle.

Toutes les conditions opératoires et les résultats sont consignés dans le Tableau (VI) suivant :

On a également conduit à titre comparatif, deux essais sans solvant organique.

On note que le présence du solvant organique permet d'accroître la quantité d'hydroquinone formée.

Tableau VI

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/ $H_2O_2$] | Solvant organique [rapport molaire solvant organique/ phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 32 | benzophénone (0,975) | oxyde de diisopropyle (0,125) | $5,1.10^{-2}$ | $0,6.10^{-2}$ | 120 mn | 100 | 45,5 | 41,5 | 1,10 |
| 33 | benzophénone (1,0) | oxyde de diisopropyle (0,25) | $5,0.10^{-2}$ | $1,35.10^{-2}$ | 90 mn | 98 | 44,0 | 40,5 | 1,09 |
| n | sans | oxyde de diisopropyle (0,125) | $5,0.10^{-2}$ | $1,45.10^{-2}$ | 230 mn | 99,5 | 38 | 43 | 0,88 |
| o | sans | oxyde de diisopropyle (0,50) | $5,0.10^{-2}$ | $1,45.10^{-2}$ | 375 mn | 96 | 34 | 36,5 | 0,93 |

EXEMPLES 34 ET 35 :

Essai comparatif p :

Dans les deux exemples suivants, on a fait appel à un composé cétonique différent par rapport aux exemples précédents.

Il s'agit de l'acétophénone.

On donne, à titre comparatif, les résultats des essais en l'absence de solvant organique (essai p).

Toutes les conditions et les résultats obtenus sont rassemblés dans le tableau (VII) suivant :

Tableau VII

HYDROXYLATION DU PHENOL PAR $H_2O_2/HClO_4$/COMPOSE CETONIQUE DE FORMULE (II)

| Réf. | Composé cétonique (II) [rapport molaire composé cétonique (II)/$H_2O_2$] | Solvant organique [rapport molaire solvant organique/phénol] | Rapport molaire $H_2O_2$/PHENOL | Rapport molaire $HClO_4/H_2O_2$ | Durée | TT | $RT_{HQ}$ | $RT_{PC}$ | Rapport HQ/PC |
|---|---|---|---|---|---|---|---|---|---|
| 34 | acétophénone (0,93) | oxyde de diisopropyle (0,12) | $5,25.10^{-2}$ | $1,15.10^{-2}$ | 16 mn | 100 | 37 | 49 | 0,76 |
| 35 | acétophénone (1,0) | oxyde de diisopropyle (0,25) | $5,1\ .10^{-2}$ | $1,20.10^{-2}$ | 90 mn | 100 | 40,5 | 47 | 0,86 |
| p | acétophénone (0,975) | sans | $5,1.10^{-2}$ | $1,25.10^{-2}$ | 2 mn | 100 | 34,5 | 49 | 0,70 |

L'examen du tableau (VII) met nettement en évidence que la présence d'un solvant organique tel que défini selon l'invention favorise la formation de l'hydroquinone.

## Revendications

1. Procédé de monohydroxylation d'un composé phénolique présentant un atome d'hydrogène libre en position para, en vue de favoriser la formation d'un composé aromatique para-dihydroxylé, par réaction du composé phénolique de départ avec le peroxyde d'hydrogène, en présence d'une quantité efficace d'un acide tort et d'un composé cétonique, ledit procédé étant caractérisé par le tait que la réaction est conduite en présence d'une quantité efficace :

   - d'un composé cétonique choisi parmi la benzophénone ou une benzophénone porteur d'un groupe électrodonneur,
   - et d'un solvant organique aprotique polaire présentant une basicité telle qu'il possède un "nombre donneur" intérieur à 25,

   et que la quantité de peroxyde d'hydrogène est telle que le rapport molaire $H_2O_2$/composé phénolique est au plus de 0,3.

2. Procédé selon la revendication 1 caractérisé par le fait que le solvant organique est un solvant organique polaire ayant une constante diélectrique supérieure ou égale à 20, de préférence, comprise entre 25 et 75.

3. Procédé selon l'une des revendications 1 et 2 caractérisé par le fait que le solvant organique polaire a un nombre donneur intérieur ou égal à 20, de préférence, compris entre 2 et 17.

4. Procédé selon l'une des revendications 1 à 3 caractérisé par le fait que le solvant organique polaire est choisi parmi les composés nitrés, les nitriles aliphatiques ou aromatiques, la tétraméthylène sulfone, le carbonate de propylène.

5. Procédé selon l'une des revendications 1 à 4 caractérisé par le fait que le solvant organique polaire est choisi parmi les solvants suivants : le nitrométhane, le nitroéthane, le nitro-1 propane, le nitro-2 propane ou leurs mélanges, le nitrobenzène, l'acétonitrile, le propionitrile, le butanenitrile, l'isobutanenitrile, le benzonitrile, le cyanure de benzyle, la tétraméthylène sulfone (sulfolane), le carbonate de propylène.

6. Procédé selon la revendication 1 caractérisé par le fait que le solvant organique est un solvant organique polaire ayant une constante diélectrique inférieure à environ 20, de préférence, comprise entre 2 et 15.

7. Procédé selon l'une des revendications 1 et 6 caractérisé par le fait que le solvant organique polaire a un nombre donneur supérieur ou égal à 15 et intérieur à 25, de préférence, compris entre 15 et 25.

8. Procédé selon l'une des revendications 1, 6 et 7 caractérisé par le fait que le solvant organique polaire est choisi parmi les éther-oxydes aliphatiques, cycloaliphatiques ou aromatiques, les esters neutres phosphoriques, le carbonate d'éthylène.

9. Procédé selon l'une des revendications 1, 6 à 8 caractérisé par le fait que le solvant organique polaire est choisi parmi les solvants suivants : l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne, le phosphate de triméthyle, le phosphate de triéthyle, le phosphate de butyle, le phosphate de triisobutyle, le phosphate de tripentyle, le carbonate d'éthylène.

10. Procédé selon l'une des revendications 1 à 9 caractérisé par le fait que le composé cétonique mis en oeuvre répond à la formule (IIa) :

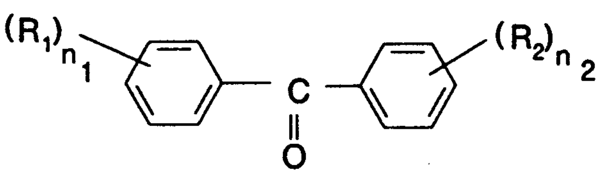

(IIa)

dans ladite formule (IIa) :

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position $\alpha$ par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

**11.** Procédé selon la revendication 10 caractérisé par le fait que le composé cétonique répond à la formule générale (IIa) dans laquelle $R_1$ et $R_2$, identiques ou différents représentent :

- des radicaux alkyle linéaires ou ramifiés ayant de 1 à 4 atomes de carbone,
- un radical phényle,
- des radicaux alkoxy $R_3$ - O dans lesquels $R_3$ représente un radical alkyle linéaire ou ramifié ayant de 1 à 4 atomes de carbone ou le radical phényle,
- un groupe hydroxyle,
- un atome de fluor.

**12.** Procédé selon la revendication 10 caractérisé par le fait que le composé cétonique répond à la formule générale (IIa) dans laquelle $R_1$ et $R_2$, identiques ou différents représentent un atome d'hydrogène ou un substituant tel que défini dans la revendication 11, de préférence en position 4,4' et $n_1$, $n_2$ identiques ou différents sont égaux à 0 ou 1.

**13.** Procédé selon la revendication 10 caractérisé par le fait que le composé cétonique répond à la formule générale (IIa) dans laquelle $R_1$ et $R_2$, identiques ou différents représentent un atome d'hydrogène ; un radical méthyle, éthyle, tert-butyle, phényle ; un radical méthoxy ou éthoxy ; un groupe hydroxyle, de préférence en position 3,3' ou 4,4'.

**14.** Procédé selon l'une des revendications 1 à 13 caractérisé par le fait que le composé cétonique mis en oeuvre est choisi parmi : la benzophénone, la méthyl-2 benzophénone, la diméthyl-2,4 benzophénone, la diméthyl-4,4' benzophénone, la diméthyl-2,2' benzophénone, la diméthoxy-4,4' benzophénone, l'hydroxy-4 benzophénone, la dihydroxy-4-4' benzophénone, le benzoyl-4 biphényle, la fluorénone.

**15.** Procédé selon l'une des revendications 1 à 14 caractérisé par le fait que le composé phénolique répond à la formule générale (I):

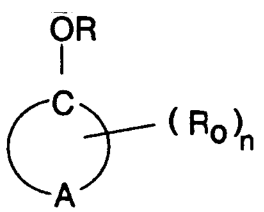

(I)

dans laquelle :

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un atome d'hydrogène ou un radical hydrocarboné ayant de 1 à 24 atomes de carbone qui peut être un radical aliphatique saturé ou insaturé, linéaire ou ramifié ou un radical cycloaliphatique saturé ou insaturé ou aromatique, monocyclique ou polycyclique,
- $R_o$ représente un ou plusieurs substituants, identiques ou différents,
- n est un nombre inférieur ou égal à 4.

**16.** Procédé selon la revendication 15 caractérisé par le fait que le composé phénolique répond à la formule (I) dans laquelle le radical R représente l'un des groupes suivants :

. un atome d'hydrogène
. un radical alkyle linéaire ou ramifié ayant de 1 à 6 atomes de carbone,
. un radical cyclohexyle,

. un radical phényle,
. un radical benzyle,

- le ou les radicaux $R_o$ représentent l'un des groupes suivants :

  . un atome d'hydrogène
  . un radical alkyle linéaire ou ramifié ayant de 1 à 6 atomes de carbone,
  . un radical cyclohexyle,
  . un radical alcényle linéaire ou ramifié ayant de 2 à 6 atomes de carbone,
  . un radical alkoxy du type $R_1$-O-, où $R_1$ représente un radical alkyle linéaire ou ramifié ayant de 1 à 6 atomes de carbone,
  . un groupe acyle ayant de 2 à 6 atomes de carbone,
  . un groupe $-COOR_2$, où $R_2$ représente un radical alkyle linéaire ou ramifié ayant de 1 à 4 atomes de carbone,
  . un atome d'halogène, de préférence, de fluor, de chlore, de brome,
  . un groupe $-CF_3$.

**17.** Procédé selon les revendications 15 ou 16 caractérisé par le fait que le composé phénolique répond à la formule (I) dans laquelle n est un nombre égal à 0, 1, 2 ou 3.

**18.** Procédé selon les revendications 15 à 17 caractérisé par le fait que le composé phénolique répond à la formule (I) dans laquelle le reste A représente :

- un radical carbocyclique aromatique monocyclique ou polycyclique avec des cycles pouvant former entre eux un système orthocondensé, la formule (I) répondant alors à la formule (Ia):

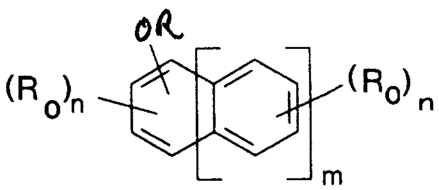

(Ia)

dans ladite formule (Ia), m représente un nombre égal à 0, 1 ou 2 et les symboles $R_o$ et n identiques ou différents ayant la signification donnée dans les revendications 15 à 17,
- un radical constitué par un enchaînement de deux ou plusieurs radicaux carbocycliques aromatiques monocycliques, la formule I répondant alors à la formule (Ib) :

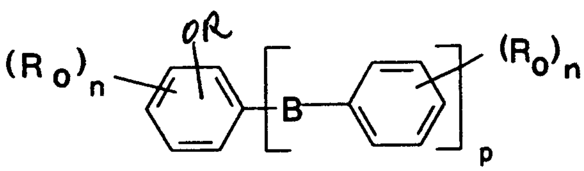

(Ib)

dans ladite formule (Ib), les symboles $R_o$ et n identiques ou différents ont la signification donnée dans les revendications 15 à 17, p est un nombre égal à 1, 2 ou 3 et B représente :
- un lien valentiel
- un radical alkylène ou alkylidène ayant de 1 à 4 atomes de carbone, de préférence, un radical méthylène ou isopropylidène,
- un des groupes suivants :
  -O-, -COO-, -OCOO-,
  $SO_2$-,

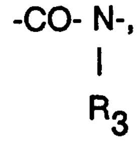

dans ces formules, $R_3$ représente un atome d'hydrogène ou un radical alkyle ayant de 1 à 4 atomes de carbone, un radical cyclohexyle ou phényle.

**19.** Procédé selon la revendication 18 caractérisé par le fait que le composé phénolique de formule (I) présente un reste A qui répond aux formules (Ia) et (Ib) dans lesquelles :

- $R_o$ représente un atome d'hydrogène, un radical alkyle ou alkoxy linéaire ou ramifié ayant de 1 à 6 atomes de carbone, de préférence, de 1 à 4 atomes de carbone un radical cyclohexyle, un radical phényle,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

**20.** Procédé selon la revendication 15 caractérisé par le fait que le composé phénolique répond à la formule générale (I'):

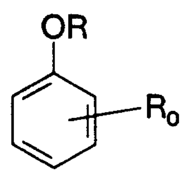

(I')

dans laquelle R et $R_o$, identiques ou différents représentent un atome d'hydrogène, un radical alkyle ayant de 1 à 4 atomes de carbone, un radical cyclohexyle, un radical phényle, et préférentiellement R représente un atome d'hydrogène et $R_o$ représente un atome d'hydrogène, un radical méthyle, un radical méthoxy.

**21.** Procédé selon l'une des revendications 1 à 20 caractérisé par le fait que le composé phénolique de formule (I) est choisi parmi : le phénol, l'anisole, l'orthocrésol, le métacrésol, le méthoxy-2 phénol.

**22.** Procédé selon l'une des revendications 1 à 21 caractérisé par le fait que le composé phénolique de formule (I) est le phénol.

**23.** Procédé selon l'une des revendications 1 à 5 caractérisé par le fait que la quantité de solvant mis en oeuvre est telle que le rapport molaire entre le nombre de moles de solvant organique et le nombre de moles de composé phénolique de formule (I) varie entre 0,1 et 2,0, de préférence, entre 0,25 et 1,0.

**24.** Procédé selon l'une des revendications 1, 6 à 9 caractérisé par le fait que la quantité de solvant mis en oeuvre est telle que le rapport molaire entre le nombre de moles de solvant organique et le nombre de moles de composé phénolique de formule (I) varie entre 0,01 et 0,25, de préférence, entre 0,025 et 0,15.

**25.** Procédé selon l'une des revendications 1 à 24 caractérisé par le fait que la quantité de composé cétonique de formule (IIa) est d'au moins $1.10^{-3}$ mole par mole de peroxyde d'hydrogène, de préférence, comprise entre $1.10^{-3}$ mole et 10 moles par mole de peroxyde d'hydrogène et encore plus préférentiellement entre 0,05 et 1,0 mole.

**26.** Procédé selon l'une des revendications 1 à 25 caractérisé par le fait que l'acide fort est un acide présentant un pka dans l'eau inférieur à - 0,1, de préférence inférieur à - 1,0.

**27.** Procédé selon l'une des revendications 1 à 26 caractérisé par le fait que l'acide fort est l'acide perchlorique ou l'acide trifluorométhanesulfonique.

**28.** Procédé selon l'une des revendications 1 à 27 caractérisé par le fait que la quantité d'acide fort est telle que le rapport $H^+/H_2O_2$ est compris entre $1.10^{-4}$ et 1,0, de préférence, entre $1.10^{-3}$ et 0,1.

**29.** Procédé selon l'une des revendications 1 à 28 caractérisé par le fait que le rapport molaire $H_2O_2$/composé phénolique de formule (I) est compris entre 0,01 et 0,3, de préférence, entre 0,05 et 0,10.

**30.** Procédé selon l'une des revendications 1 à 29 caractérisé par le fait que l'on opère à une température comprise entre 45°C et 150°C, de préférence, entre 45°C et 75°C.

## Claims

**1.** A process for the monohydroxylation of a phenolic compound having a hydrogen atom free in the para position, for the purpose of promoting the formation of a para-dihydroxylated aromatic compound, by reacting the initial phenolic compound with hydrogen peroxide in the presence of an effective amount of a strong acid and ketonic compound, said process being characterised in that the reaction is carried out in the presence of an effective amount

- of a ketonic compound selected from benzophenone or a benzophenone bearing an electron donor group,
- and a polar aprotic organic solvent having a basicity such that it has a "donor number" of less than 25,

and that the amount of hydrogen peroxide is such that the $H_2O_2$/phenolic compound molar ratio is a maximum of 0.3.

**2.** A process according to Claim 1, characterised in that the organic solvent is a polar organic solvent with a dielectric constant above or equal to 20, preferably between 25 and 75.

**3.** A process according to either Claim 1 or Claim 2, characterised by the fact that the polar organic solvent has a donor number less than or equal to 20, preferably between 2 and 17.

**4.** A process according to one of Claims 1 to 3, characterised by the fact that the polar organic solvent is selected from nitrated compounds, aliphatic or aromatic nitriles, tetramethylene sulphone, propylene carbonate.

**5.** A process according to one of Claims 1 to 4, characterised by the fact that the polar organic solvent is selected from the following solvents: nitromethane, nitroethane, 1-nitropropane, 2-nitropropane or mixtures thereof, nitrobenzene, acetonitrile, propionitrile, butanenitrile, isobutanenitrile, benzonitrile, benzyl cyanide, tetramethylene sulphone (sulpholane), propylene carbonate.

**6.** A process according to Claim 1, characterised by the fact that the organic solvent is a polar organic solvent with a dielectric constant of less than about 20, preferably between 2 and 15.

**7.** A process according to one of Claims 1 and 6, characterised by the fact that the polar organic solvent has a donor number above or equal to 15 and less than 25, preferably between 15 and 25.

**8.** A process according to one of Claims 1, 6 and 7, characterised by the fact that the polar organic solvent is selected from the aliphatic, cycloaliphatic or aromatic ether-oxides, phosphoric neutral esters, ethylene carbonate.

**9.** A process according to one of Claims 1, 6 to 8, characterised by the fact that the polar organic solvent is selected from the following solvents: diethyl oxide, dipropyl oxide, diisopropyl oxide, dibutyl oxide, methyl t-butyl ether, dipentyl oxide, diisopentyl oxide, ethyleneglycol dimethylether (or 1,2-dimethoxyethane), diethylene glycol dimethylether (or 1,5-dimethoxy-3-oxapentane), dioxane, tetrahydrofuran, trimethyl phosphate, triethyl phosphate, butyl phosphate, triisobutyl phosphate, tripentyl phosphate, ethylene carbonate.

**10.** A process according to one of Claims 1 to 9, characterised by the fact that the ketonic compound used corresponds to the formula (IIa):

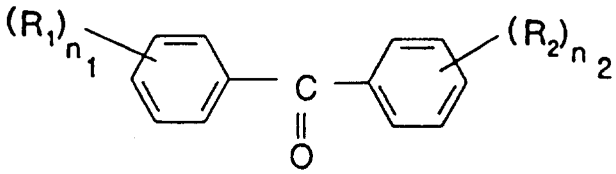

(IIa)

in which formula (IIa) :

- $R_1$ and $R_2$ which may be the same or different represent a hydrogen atom or an electron-donor group,
- $n_1$, $n_2$ which may be the same or different is a number equal to 0, 1, 2 or 3,
- the two carbon atoms disposed in the $\alpha$ position to the two carbon atoms bearing the -CO group can be linked together by a valency linkage or by a $-CH_2-$ group thus forming a ketonic cycle which can be saturated but also unsaturated.

**11.** A process according to Claim 10, characterised by the fact that the ketonic compound corresponds to general formula (IIa) in which $R_1$ and $R_2$ which may be identical or different represent:

- linear or branched alkyl radicals with 1 to 4 carbon atoms,
- a phenyl radical,
- $R_3$ - 0 alkoxy radicals in which $R_3$ represents a linear or branched alkyl radical with 1 to 4 carbon atoms, or the phenyl radical,
- a hydroxyl group,
- a fluorine atom.

**12.** A process according to Claim 10, characterised by the fact that the ketonic compound corresponds to general formula (11a) in which $R_1$ and $R_2$ which are the same or different represent a hydrogen atom or a substituent such as defined in Claim 11, preferably in position 4,4', and $n_1$, $n_2$ which are the same or different are equal to 0 or 1.

**13.** A process according to Claim 10, characterised by the fact that the ketonic compound corresponds to general formula (IIa) in which $R_1$ and $R_2$ which are identical or different represent a hydrogen atom, a methyl, ethyl, terbutyl, or phenyl radical; a methoxy or ethoxy radical; a hydroxyl group, preferably in position 3,3' or 4,4'.

**14.** A process according to one of Claims 1 to 13, characterised in that the ketonic compound used is selected from : benzophenone, 2-methylbenzophenone, 2,4-dimethylbenzophenone, 4,4'-dimethylbenzophenone, 2,2'-dimethylbenzophenone, 4,4'-dimethoxybenzophenone, 4-hydroxybenzophenone, 4,4'dihydroxybenzophenone, 4-benzoylbiphenyl, fluorenone.

**15.** A process according to one of Claims 1 to 14, characterised by the fact that the phenolic compound corresponds to the general formula (I) :

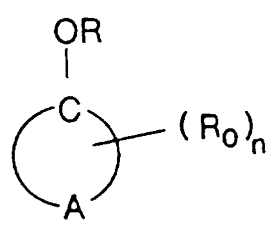

(I)

wherein:

- A symbolises a residue of a monocyclic or polycyclic aromatic carbocyclic radical or a divalent radical constituted by a chain of two or more monocyclic aromatic carbocyclic radicals,
- R represents a hydrogen atom or a hydrocarbon radical with 1 to 24 carbon atoms which can be a saturated

or unsaturated, linear or branched aliphatic radical, or a saturated or unsaturated, or aromatic, monocyclic or polycyclic cycloaliphatic radical,

- $R_o$ represents one or a plurality of substituents which may be the same or different,
- n is a number less than or equal to 4.

**16.** A process according to Claim 15, characterised by the fact that the phenolic compound corresponds to formula (I) wherein the radical R represents one of the following groups:

. a hydrogen atom,
. a linear or branched alkyl radical with 1 to 6 carbon atoms,
. a cyclohexyl radical,
. a phenyl radical,
. a benzyl radical,

- the $R_o$ radical(s) represents/represent one of the following groups:

  . a hydrogen atom
  . a linear or branched alkyl radical with 1 to 6 carbon atoms,
  . a cyclohexyl radical
  . a linear or branched alkenyl radical with 2 to 6 carbon atoms,
  . an alkoxy radical of the $R_1$-O- type wherein $R_1$ represents a linear or branched alkyl radical with 1 to 6 carbon atoms,
  . an acyl group with 2 to 6 carbon atoms,
  . a $-COOR_2$-group, where $R_2$ represents a linear or branched alkyl radical with 1 to 4 carbon atoms,
  . a halogen atom, preferably fluorine, chlorine, bromine
  . a $-CF_3$ group.

**17.** A process according to Claim 15 or 16 characterised by the fact that the phenolic compound corresponds to formula (I) wherein n is a number equal to 0, 1, 2 or 3.

**18.** A process according to Claims 15 to 17, characterised by the fact that the phenolic compound corresponds to formula (I) wherein the residue A represents:

- a monocyclic or polycyclic aromatic carbocyclic radical with cycles capable of forming between them an orthocondensed system, formula (I) then corresponding to formula (Ia):

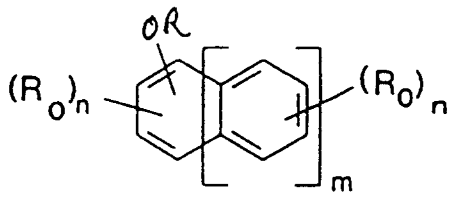

(Ia)

  in which formula (Ia), m represents a number equal to 0, 1 or 2 and the symbols $R_o$ and n which may be the same or different having the meaning given in Claims 15 to 17,

- a radical constituted by a chain of two or more monocyclic aromatic carbocyclic radicals, formula (I) then corresponding to formula (Ib) :

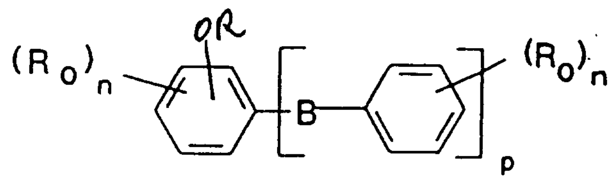

(Ib)

in which formula (Ib), the symbols $R_o$ and n which may be the same or different have the meaning given in Claims 15 to 17, p is a number equal to 1, 2 or 3 and B represents :

- a valency linkage
- an alkylene or alkylidene radical with 1 to 4 carbon atoms, preferably a methylene or isopropylidene radical,
- one of the following groups :

  -O-, -COO-, -OCOO-

  $-SO_2-$ ,

-CO- N-,
|
$R_3$

in these formulae, $R_3$ represents a hydrogen atom or an alkyl radical with 1 to 4 carbon atoms. a cyclohexyl radical or a phenyl radical.

**19.** A process according to Claim 18, characterised by the fact that the phenolic compound of formula (I) has a residue A which corresponds to formulae (Ia) and (Ib) wherein:

- $R_o$ represents a hydrogen atom, a linear or branched alkyl or alkoxy radical with 1 to 6 carbon atoms, preferably 1 to 4 carbon atoms, a cyclohexyl radical, a phenyl radical,
- B symbolises a valency linkage, an alkylene or alkylidene radical with 1 to 4 carbon atoms or an oxygen atom,
- m is equal to 0 or 1,
- n is equal to 0, 1 or 2,
- p is equal to 0 or 1.

**20.** A process according to Claim 15, characterised by the fact that the phenolic compound corresponds to the general formula (I'):

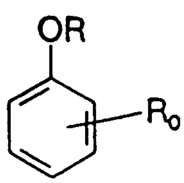

(I')

wherein R and $R_o$ which may be the same or different represent a hydrogen atom, an alkyl radical with 1 to 4 carbon atoms, a cyclohexyl radical, a phenyl radical, and preferably R represents a hydrogen atom and $R_o$ represents a hydrogen atom, a methyl radical, a methoxy radical.

**21.** A process according to one of Claims 1 to 20, characterised by the fact that the phenolic compound of formula (I) is selected from: phenol, anisol, orthocresol, metacresol, 2-methoxy phenol.

**22.** A process according to one of Claims 1 to 21, characterised by the fact that the phenolic compound of formula (I)

is phenol.

23. A process according to one of Claims 1 to 5, characterised by the fact that the amount of solvent used is such that the molar ratio between the number of moles of the organic solvent and the number of moles of the phenolic compound of formula (I) varies between 0.1 and 2.0, preferably between 0.25 and 1.0.

24. A process according to one of Claims 1, 6 to 9, characterised by the fact that the amount of solvent used is such that the molar ratio between the number of moles of the organic solvent and the number of moles of the phenolic compound of formula (I) varies between 0.01 and 0.25, preferably between 0.025 and 0.15.

25. A process according to one of Claims 1 to 24, characterised by the fact that the amount of ketonic compound of formula (IIa) is at least $1.10^{-3}$ moles per mole of hydrogen peroxide, preferably between $1.10^{-3}$ mole and 10 moles per mole of hydrogen peroxide, and still more preferably between 0.05 and 1.0 mole.

26. A process according to one of Claims 1 to 25, characterised by the fact that the strong acid is an acid with a pka in water of less than -0.1, preferably less than -1.0.

27. A process according to one of Claims 1 to 26, characterised by the fact that the strong acid is perchloric acid or trifluoro-methane sulphonic acid.

28. A process according to one of Claims 1 to 27, characterised by the fact that the amount of strong acid is such that the ratio $H^+/H_2O_2$ is between $1.10^{-4}$ and 1.0, preferably between $1.10^{-3}$ and 0.1.

29. A process according to one of Claims 1 to 28, characterised by the fact that the molar ratio $H_2O_2$/phenolic compound of formula (I) is between 0.01 and 0.3, preferably between 0.05 and 0.10.

30. A process according to one of Claims 1 to 29, characterised by the fact that the process is carried out at a temperature of between 45°C and 150°, preferably of between 45°C and 75°C.

**Patentansprüche**

1. Verfahren zur Monohydroxylierung einer Phenolverbindung, die ein freies Wasserstoffatom in der para-Position aufweist, um die Bildung einer para-dihydroxylierten aromatischen Verbindung zu begünstigen, mittels Reaktion der Phenolausgangsverbindung mit Wasserstoffperoxid in Gegenwart einer wirksamen Menge einer starken Säure und einer Ketoverbindung, wobei das Verfahren dadurch gekennzeichnet ist, daß die Reaktion in Gegenwart einer wirksamen Menge:

   - einer Ketoverbindung, ausgewählt aus Benzophenon oder einem Benzophenon mit einer Elektronendonorgruppe;
   - und eines polaren, aprotischen organischen Lösungsmittels mit einer solchen Basizität, daß es eine „Donorzahl" von weniger als 25 aufweist,

   durchgeführt wird und daß die Menge an Wasserstoffperoxid derart ist, daß das $H_2O_2$/Phenolverbindung-Molverhältnis höchstens 0,3 beträgt.

2. Verfahren nach Anspruch 1, dadurch gekennzeichnet, daß das organische Lösemittel ein polares organisches Lösungsmittel mit einer Dielektrizitätskonstante größer oder gleich 20, vorzugsweise zwischen 25 und 75, ist.

3. Verfahren nach Anspruch 1 oder 2, dadurch gekennzeichnet, daß das polare organische Lösemittel eine Donorzahl unterhalb von oder gleich 20, vorzugsweise zwischen 2 und 17, aufweist.

4. Verfahren nach einem der Ansprüche 1 bis 3, dadurch gekennzeichnet, daß das polare organische Lösungsmittel ausgewählt ist aus nitrierten Verbindungen, aliphatischen oder aromatischen Nitrilen, Tetramethylensulfon und Propylencarbonat.

5. Verfahren nach einem der Ansprüche 1 bis 4, dadurch gekennzeichnet, daß das polare organische Lösungsmittel ausgewählt ist aus den folgenden Lösungsmitteln: Nitromethan, Nitroethan, 1-Nitropropan, 2-Nitropropan oder deren Mischungen, Nitrobenzol, Acetonitril, Propionitril, Butannitril, Isobutannitril, Benzonitril, Benzylcyanid, Tetramethylensulfon (Sulfolan) und Propylencarbonat.

6. Verfahren nach Anspruch 1, dadurch gekennzeichnet, daß das organische Lösungsmittel ein polares organisches Lösungsmittel mit einer Dielektrizitätskonstante von weniger als etwa 20, vorzugsweise zwischen 2 und 15, ist.

7. Verfahren nach einem der Ansprüche 1 und 6, dadurch gekennzeichnet, daß das polare organische Lösungsmittel eine Donorzahl oberhalb oder gleich 15 und unterhalb von 25, vorzugsweise zwischen 15 und 25, aufweist.

8. Verfahren nach einem der Ansprüche 1, 6 und 7, dadurch gekennzeichnet, daß das polare organische Lösungsmittel ausgewählt ist aus aliphatischen, cycloaliphatischen oder aromatischen Etheroxiden, neutralen Phosphorestern und Ethylencarbonat.

9. Verfahren nach einem der Ansprüche 1, 6 bis 8, dadurch gekennzeichnet, daß das polare organische Lösungsmittel ausgewählt ist aus den folgenden Lösungsmitteln: Diethyloxid, Dipropyloxid, Diisopropyloxid, Dibutyloxid, Methyltertiobutylether, Dipentyloxid, Diisopentyloxid, Ethylenglykoldimethylether (oder 1,2-Dimethoxyethan), Diethylenglykoldimethylether (oder 1,5-Dimethoxy-3-oxapentan), Dioxan, Tetrahydrofuran, Trimethylphosphat, Triethylphosphat, Butylphosphat, Triisobutylphosphat, Tripentylphosphat und Ethylencarbonat.

10. Verfahren nach einem der Ansprüche 1 bis 9, dadurch gekennzeichnet, daß die eingesetzte Ketoverbindung der Formel (IIa) entspricht:

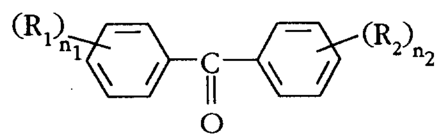

(IIa)

wobei in der Formel (IIa):

- $R_1$ und $R_2$, identisch oder verschieden, ein Wasserstoffatom oder eine Elektronendonorgruppe darstellen;
- $n_1$ und $n_2$, identisch oder verschieden, eine ganze Zahl 0, 1, 2 oder 3 sind;
- gegebenenfalls die zwei Kohlenstoffatome, die sich in $\alpha$-Position zu den zwei Kohlenstoffatomen mit der CO-Gruppe befinden, zusammen über eine Valenzbindung oder eine $CH_2$-Gruppe miteinander verbunden sein und somit einen Ketonring bilden können, der gesättigt, jedoch auch ungesättigt sein kann.

11. Verfahren nach Anspruch 10, dadurch gekennzeichnet, daß die Ketoverbindung der allgemeinen Formel (IIa) entspricht, in der $R_1$ und $R_2$, identisch oder verschieden, darstellen:

- lineare oder verzweigte Alkylreste mit 1 bis 4 Kohlenstoffatomen,
- einen Phenylrest,
- Alkoxyreste $R_3$-O, in denen $R_3$ einen linearen oder verzweigten Alkylrest mit 1 bis 4 Kohlenstoffatomen oder einen Phenylrest darstellt,
- eine Hydroxylgruppe,
- ein Fluoratom.

12. Verfahren nach Anspruch 10, dadurch gekennzeichnet, daß die Ketoverbindung der allgemeinen Formel (IIa) entspricht, in der $R_1$ und $R_2$, identisch oder verschieden, ein Wasserstoffatom oder einen Substituenten, wie in Anspruch 11 definiert, vorzugsweise in 4,4'-Position, darstellen und wobei $n_1$ und $n_2$, identisch oder verschieden, gleich 0 oder 1 sind.

13. Verfahren nach Anspruch 10, dadurch gekennzeichnet, daß die Ketoverbindung der allgemeinen Formel (IIa) entspricht, in der $R_1$ und $R_2$, identisch oder verschieden, ein Wasserstoffatom; einen Methyl-, Ethyl-, tert.-Butyl- oder Phenylrest; einen Methoxy- oder Ethoxyrest; eine Hydroxylgruppe, vorzugsweise in 3,3'-Position oder 4,4'-Position, darstellen.

14. Verfahren nach einem der Ansprüche 1 bis 13, dadurch gekennzeichnet, daß die eingesetzte Ketoverbindung ausgewählt ist aus: Benzophenon, 2-Methylbenzophenon, 2,4-Dimethylbenzophenon, 4,4'-Dimethylbenzophenon, 2,2'-Dimethylbenzophenon, 4,4'-Dimethoxybenzophenon, 4-Hydroxybenzophenon, 4,4'-Dihydroxybenzophenon, 4-Benzoylbiphenyl und Fluorenon.

**15.** Verfahren nach einem der Ansprüche 1 bis 14, dadurch gekennzeichnet, daß die Phenolverbindung der allgemeinen Formel (I) entspricht:

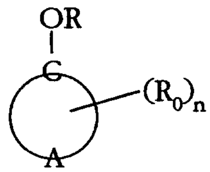

(I)

in der:

- A einen monocyclischen oder polycyclischen, aromatischen carbocyclischen Rest oder einen divalenten Rest darstellt, der aus einer Verkettung von zwei oder mehreren monocyclischen, aromatischen carbocyclischen Resten besteht;
- R ein Wasserstoffatom oder einen Kohlenwasserstoffrest mit 1 bis 24 Kohlenstoffatomen darstellt, der ein gesättigter oder ungesättigter, linearer oder verzweigter aliphatischer Rest oder ein gesättigter oder ungesättigter, cycloaliphatischer oder monocyclischer oder polycyclischer, aromatischer Rest sein kann;
- $R_0$ einen oder mehreren Substituenten, identisch oder verschieden, darstellt;
- n eine ganze Zahl kleiner oder gleich 4 ist.

**16.** Verfahren nach Anspruch 15, dadurch gekennzeichnet, daß die Phenolverbindung der Formel (I) entspricht, in der der Rest R eine der folgenden Gruppen darstellt:

- ein Wasserstoffatom,
- einen linearen oder verzweigten Alkylrest mit 1 bis 6 Kohlenstoffatomen,
- einen Cyclohexylrest,
- einen Phenylrest,
- einen Benzylrest,

- einen oder mehrere Reste $R_0$, wobei $R_0$ jeweils eine der folgenden Gruppen darstellt:
  - ein Wasserstoffatom,
  - einen linearen oder verzweigten Alkylrest mit 1 bis 6 Kohlenstoffatomen,
  - einen Cyclohexylrest,
  - einen linearen oder verzweigten Alkenylrest mit 2 bis 6 Kohlenstoffatomen,
  - einen Alkoxyrest vom Typ $R_1$-O-, wobei $R_1$ einen linearen oder verzweigten Alkylrest mit 1 bis 6 Kohlenstoffatomen darstellt,
  - eine Acylgruppe mit 2 bis 6 Kohlenstoffatomen,
  - eine Gruppe $-COOR_2$, wobei $R_2$ einen linearen oder verzweigten Alkylrest mit 1 bis 4 Kohlenstoffatomen bezeichnet,
  - ein Halogenatom, vorzugsweise ein Fluor-, Chlor- oder Bromatom,
  - eine $CF_3$-Gruppe.

**17.** Verfahren nach Anspruch 15 oder 16, dadurch gekennzeichnet, daß die Phenolverbindung der Formel (I) entspricht, in der n eine ganze Zahl 0, 1, 2 oder 3 ist.

**18.** Verfahren nach einem der Ansprüche 15 bis 17, dadurch gekennzeichnet, daß die Phenolverbindung der Formel (I) entspricht, in der der Rest A darstellt:

- einen monocyclischen oder polycyclischen, aromatischen carbocyclischen Rest mit Ringen, die untereinander ein orthokondensiertes System darstellen können wobei die Formel (I) dann der Formel (Ia) entspricht:

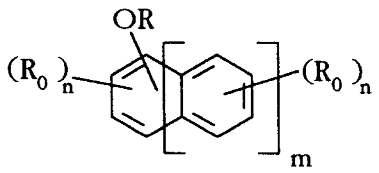

(Ia)

wobei in der Formel (Ia) m eine ganze Zahl 0, 1 oder 2 darstellt und die Symbole $R_0$ und n, jeweils identisch oder verschieden, die zuvor in den Ansprüchen 15 bis 17 angegebene Bedeutung haben;

- einen Rest, der aus einer Verkettung von 2 oder mehreren monocyclischen, aromatischen carbocyclischen Resten besteht, wobei die Formel (I) dann der Formel (Ib) entspricht:

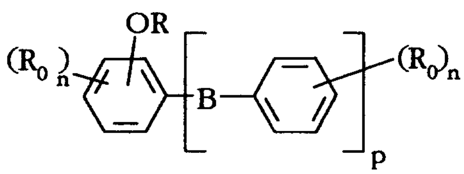

(Ib)

wobei in der Formel (Ib) die Symbole $R_0$ und n, jeweils identisch oder verschieden, die zuvor in den Ansprüchen 15 bis 17 angegebene Bedeutung haben, p eine ganze Zahl von 1, 2 oder 3 ist und B darstellt:

- eine Valenzbindung,
- einen Alkylen- oder Alkylidenrest mit 1 bis 4 Kohlenstoffatomen, vorzugsweise einen Methylen- oder Isopropylidenrest,
- eine der folgenden Gruppen
  -O-, -COO-, -OCOO-, $-SO_2-$,

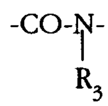

wobei in diesen Formeln $R_3$ ein Wasserstoffatom oder einen Alkylrest mit 1 bis 4 Kohlenstoffatomen, einen Cyclohexylrest oder einen Phenylrest darstellt.

**19.** Verfahren nach Anspruch 18, dadurch gekennzeichnet, daß die Phenolverbindung der Formel (I) einen Rest A darstellt, der den Formeln (Ia) und (Ib) entspricht, in denen:

- $R_0$ ein Wasserstoffatom, einen linearen oder verzweigten Alkyl- oder Alkoxyrest mit 1 bis 6 Kohlenstoffatomen, vorzugsweise mit 1 bis 4 Kohlenstoffatomen, einen Cyclohexylrest, einen Phenylrest darstellt,
- B eine Valenzbindung, einen Alkylen- oder Alkylidenrest mit 1 bis 4 Kohlenstoffatomen oder ein Sauerstoffatom darstellt,
- m gleich 0 oder 1 ist,
- n gleich 0, 1 oder 2 ist,
- p gleich 0 oder 1 ist.

**20.** Verfahren nach Anspruch 15, dadurch gekennzeichnet, daß die Phenolverbindung der allgemeinen Formel (I') entspricht:

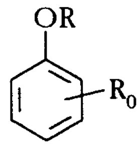

(I')

wobei in der Formel (I') R und $R_0$, identisch oder verschieden, ein Wasserstoffatom, einen Alkylrest mit 1 bis 4 Kohlenstoffatomen, einen Cyclohexylrest, einen Phenylrest darstellen und vorzugsweise R ein Wasserstoffatom darstellt und $R_0$ ein Wasserstoffatom, einen Methylrest oder einen Methoxyrest darstellt.

**21.** Verfahren nach einem der Ansprüche 1 bis 20, dadurch gekennzeichnet, daß die Phenolverbindung der Formel (I) ausgewählt ist aus: Phenol, Anisol, Orthokresol, Metakresol, 2-Methoxyphenol.

**22.** Verfahren nach einem der Ansprüche 1 bis 21, dadurch gekennzeichnet, daß die Phenolverbindung der Formel (I) Phenol ist.

**23.** Verfahren nach einem der Ansprüche 1 bis 5, dadurch gekennzeichnet, daß die Menge an eingesetztem Lösemittel derart ist, daß das Molverhältnis zwischen der Molanzahl des organischen Lösemittels und der Molanzahl der Phenolverbindung der Formel (I) zwischen 0,1 und 2,0, vorzugsweise zwischen 0,25 und 1,0, liegt.

**24.** Verfahren nach einem der Ansprüche 1, 6 bis 9, dadurch gekennzeichnet, daß die Menge an eingesetztem Lösemittel so gewählt ist, daß das Molverhältnis zwischen der Molzahl des organischen Lösemittels und der Molzahl der Phenolverbindung der Formel (I) zwischen 0,01 und 0,25, vorzugsweise zwischen 0,025 und 0,15, liegt.

**25.** Verfahren nach einem der Ansprüche 1 bis 24, dadurch gekennzeichnet, daß die Menge der Ketoverbindung der Formel (IIa) mindestens $1 \cdot 10^{-3}$ mol pro Mol Wasserstoffperoxid, vorzugsweise zwischen $1 \cdot 10^{-3}$ mol und 10 mol pro Mol Wasserstoffperoxid, insbesondere zwischen 0,05 und 1,0 mol, liegt.

**26.** Verfahren nach einem der Ansprüche 1 bis 25, dadurch gekennzeichnet, daß die starke Säure eine Säure mit einem $pK_a$-Wert in Wasser von weniger als -0,1, vorzugsweise von weniger als -1,0, ist.

**27.** Verfahren nach einem der Ansprüche 1 bis 26, dadurch gekennzeichnet, daß die starke Säure Perchlorsäure oder Trifluormethansulfonsäure ist.

**28.** Verfahren nach einem der Ansprüche 1 bis 27, dadurch gekennzeichnet, daß die Menge an starker Säure so ausgewählt ist, daß das $H^+/H_2O_2$-Verhältnis zwischen $1 \cdot 10^{-4}$ und 1,0, vorzugsweise zwischen $1 \cdot 10^{-3}$ und 0,1, liegt.

**29.** Verfahren nach einem der Ansprüche 1 bis 28, dadurch gekennzeichnet, daß das Molverhältnis $H_2O_2$/Phenolverbindung der Formel (I) zwischen 0,01 und 0,3, vorzugsweise zwischen 0,05 und 0,10, liegt.

**30.** Verfahren nach einem der Ansprüche 1 bis 29, dadurch gekennzeichnet, daß man bei einer Temperatur zwischen 45 °C und 150 °C, vorzugsweise zwischen 45 °C und 75 °C, verfährt.